(19) Europäisches Patentamt
European Patent Office
Office européen des brevets

(11) EP 2 931 243 B1

(12) **EUROPÄISCHE PATENTSCHRIFT**

(45) Veröffentlichungstag und Bekanntmachung des Hinweises auf die Patenterteilung:
**26.02.2020 Patentblatt 2020/09**

(21) Anmeldenummer: **13792336.3**

(22) Anmeldetag: **14.11.2013**

(51) Int Cl.:
***A61K 8/86*** *(2006.01)* ***A61K 8/81*** *(2006.01)*
***A61Q 5/08*** *(2006.01)* ***A61Q 5/10*** *(2006.01)*
***A61K 8/34*** *(2006.01)* ***A61K 8/92*** *(2006.01)*
***A61K 8/39*** *(2006.01)* ***A61K 8/898*** *(2006.01)*

(86) Internationale Anmeldenummer:
**PCT/EP2013/073787**

(87) Internationale Veröffentlichungsnummer:
**WO 2014/090499 (19.06.2014 Gazette 2014/25)**

(54) **REDUZIERUNG DES AMMONIAKGERUCHS IN MITTELN ZUM FÄRBEN UND/ODER AUFHELLEN VON HAAREN**

REDUCTION OF AMMONIA ODOR IN AGENTS FOR DYEING AND/OR BLEACHING HAIR

RÉDUCTION DE L'ODEUR D'AMMONIAC DANS DES AGENTS DESTINÉS À COLORER ET/OU ECLAIRCIR LES CHEVEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2012 DE 102012223205**

(43) Veröffentlichungstag der Anmeldung:
**21.10.2015 Patentblatt 2015/43**

(73) Patentinhaber: **Henkel AG & Co. KGaA**
**40589 Düsseldorf (DE)**

(72) Erfinder:
- **NEUBA, Constanze**
  **41516 Grevenbroich (DE)**
- **JANßEN, Frank**
  **51103 Köln (DE)**
- **UCHIDA, Kota**
  **Chiba (JP)**
- **MACHIDA, Shoji**
  **Tokyo, 123-0872 (JP)**

(56) Entgegenhaltungen:
**EP-A1- 1 832 273** **EP-A1- 1 878 469**
**EP-A1- 2 308 564** **EP-A2- 1 048 290**
**EP-A2- 1 714 637** **DE-A1-102004 039 181**
**DE-A1-102009 003 002**

- **DATABASE GNPD [Online] MINTEL; 1. November 2012 (2012-11-01), L'Oréal: "Hair Colourant", XP002729962, Database accession no. 1922137**
- **Nn: "Loreal Sublime Mousse Hair Color", YouTube, 10 March 2011 (2011-03-10), pages 1-3, XP055441377, Retrieved from the Internet: URL:https://www.youtube.com/watch?v=7FZnEIYI6wI [retrieved on 2018-01-16]**

Bemerkungen:
Die Akte enthält technische Angaben, die nach dem Eingang der Anmeldung eingereicht wurden und die nicht in dieser Patentschrift enthalten sind.

EP 2 931 243 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gegenstand der vorliegenden Erfindung sind Mittel zum Färben und/oder Aufhellen von keratinischen Fasern, insbesondere menschlichen Haaren, enthaltend in einem kosmetischen Träger eine spezielle Kombination aus mindestens einem Oxidationsfarbstoffvorprodukt, Ammoniak, mindestens einem nichtionischen Emulgator vom Typ der ethoxylierten Fettalkohole mit einem Ethoxylierungsgrad von 80 bis 120, mindestens einem ethoxylierten Fettalkohol mit einem Ethoxylierungsgrad von 30, mindestens einem zwitterionischen Polymer und zusätzlich einem oder mehreren Fettalkoholen aus der Gruppe Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol). Es besteht die Maßgabe, dass das Mittel keine Carbonate enthält.

Weiterhin betrifft die Erfindung die Verwendung dieser speziellen Kombination zur Reduktion des Ammoniak-Geruchs vor, während und nach dem Färbe- und/oder Aufhellvorgang.

Zur Bereitstellung farbverändernder kosmetischer Mittel, insbesondere für keratinische Fasern, wie beispielsweise Haare, kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Die Oxidationsfärbemittel zeichnen sich durch hervorragende, lang anhaltende Färbeergebnisse aus. Neben der Färbung ist auch das Aufhellen bzw. Blondieren der eigenen Haarfarbe der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt.

Um eine zufriedenstellende Färbe- und Aufhellleistung zu entfalten, benötigen oxidative Färbe- bzw. Aufhellmittel bei der Anwendung in der Regel einen alkalischen pH-Wert, insbesondere bei pH-Werten zwischen 8,5 und 10,5 werden optimale Ergebnisse erzielt.

Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak - ebenfalls in stärkerem Ausmaß als alle anderen handelsüblichen Alkalisierungsmittel - als Penetrations- bzw. Penetrationshilfsmittel.

Aus diesen Gründen werden bei Einsatz von Ammoniak in oxidativen Färbemitteln im Vergleich zu anderen Alkalisierungsmitteln (wie beispielsweise Kalium- oder Natriumhydroxid, Alkanolaminen oder Carbonaten wie Natriumcarbonat oder Kaliumcarbonat) intensivere Färbungen und signifikant bessere Grauabdeckungen erhalten.

Bedingt durch die von Anfang an höheren Farbintensitäten sind auch die Echtheitseigenschaften der mit Hilfe von Ammoniak erzeugten Haarfärbungen besser. Insbesondere besitzen gefärbte Haare dann die besten Waschechtheiten, wenn Ammoniak als Alkalisierungsmittel gewählt wurde.

Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher oxidativer Färbemittel verwendet wird.

Aus der Literatur sind bereits umfangreiche Bestrebungen zur Reduzierung des Ammoniak-Geruchs bekannt. Hierbei bestehen zur Minimierung des Geruchs drei prinzipielle Möglichkeiten: Als erste Möglichkeit nennt die Literatur die Variation des Alkalisierungsmittels und damit den teilweisen oder vollständigen Ersatz von Ammoniak durch geruchslose Alternativen.

So existiert beispielsweise eine Vielzahl von Rezepturen, welche ein Gemisch von Ammoniak und Monoethanolamin oder ausschließlich Monoethanolamin als Alkalisierungsmittel einsetzen. Eine Reduktion des Ammoniak-Gehaltes hat jedoch generell eine schlechtere Penetration der Farbstoffe in das Haar hinein zur Folge, was sich wie zuvor beschrieben insbesondere in schlechteren Grauabdeckungen und einer schlechteren Waschechtheiten niederschlägt. Wenn die Entwicklung von besonders haltbaren Nuancen im Fokus steht, ist der Einsatz von Monoethanolamin deshalb keine Option.

Die WO 2006060570 und WO 2006060565 schlagen für die Bereitstellung von oxidativen Färbemitteln mit geringer Geruchsbelastung den Einsatz von Carbonaten oder Carbonatquellen als Alkalisierungsmittel vor. Es ist jedoch ebenfalls literaturbekannt, dass Carbonate in Kombination mit Oxidationsmitteln die Haare in stärkerem Ausmaß schädigen können. Die durch die Carbonate hervorgerufene zusätzliche Schädigung des Haares mag bei Anwendung des Färbemittels auf unbehandeltem bzw. ungeschädigtem Haar wenig problematisch sein, kann sich jedoch bei Personen, die ihr Haar regelmäßig färben bzw. blondieren, zu schweren, kumulativen Schäden aufsummieren. Wird eine stärkere Aufhellung und/oder regelmäßige Färbung gewünscht, stellt der Einsatz von Carbonaten daher ebenfalls keine gangbare Alternative dar.

Eine zweite prinzipielle Möglichkeit zur Reduktion des Ammoniak-Geruchs besteht im Zusatz von speziellen

Parfumstoffen, welche den Ammoniak-Geruch überdecken sollen. Dieser Weg wird beispielsweise in der WO 2005/110499 beschritten. Parfumstoffe können jedoch unter den alkalischen Lagerbedingungen instabil sein, so dass die Gefahr besteht, dass die Duftstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft.

Eine dritte prinzipielle Möglichkeit zur Reduktion des Ammoniakgeruchs besteht in einer Optimierung der Formulierung. Hierbei gilt es, die Träger-Bestandteile der Formulierung so auszuwählen, dass diese einen optimalen Rückhalt des Ammoniaks in der Formulierung gewährleisten und auf diese Weise seinen Geruch minimieren. Es ist jedoch ebenfalls bekannt, dass die Formulierung, die in ihr enthaltenen Fettstoffe, ihre Emulgatoren, Tenside und ihre Viskosität wesentlichen Einfluss auf die Färbeleistung nehmen. Bei der Modifikation der Formulierung muß eine Verschlechterung der Färbeleistung daher auf jeden Fall vermieden werden.

Beispielsweise schlägt die JP 2007191459 zur Reduktion des Ammoniak-Geruchs in Haarfärbemitteln den Einsatz von kationischen Tensiden, Phophateestern und aliphatischen Alkoholen vor. Die JP 2003040750 offenbart, dass der Ammoniak-Geruch in Blondiermittel dann besonders gering ist, wenn den Mitteln mindestens 5 % einer kristallinen Komponente zugesetzt wird.

Ein im November 2012 in Israel in den Markt gebrachtes Haarfärbeprodukt der Marke "Sublime Mousse" von L'Oreal (Produkt-Datenbank Mintel, Record ID 1922137) umfasst drei getrennt konfektionierte Komponenten, eine Färbecreme (Liquid to Mousse Colorant), eine Oxidationsmittelzubereitung (Liquid to Mousse Developer) und einen Conditioner (Healthy shine Conditioner). Die Färbecreme (Liquid to Mousse Colorant) enthält Oxidationsfarbstoffe, Ammoniak und einen ethoxylierten Fettalkohol mit einem Ethoxylierungsgrad von 100. Die Oxidationsmittelzubereitung (Liquid to Mousse Developer) enthält ein zwitterionisches Polymer.

EP 1878469 A1 beschreibt Mittel zum Färben oder Aufhellern von Haaren, welche vor der Anwendung durch Vermischen von einer Oxidationsmittelzubereitung mit einem Gel-Netzwerk-Verdicker-System erhalten werden.

Insbesondere die dauerhafte Geruchsminimierung über die gesamte Anwendungsdauer ist nur sehr schwierig zu erreichen. Die Zeitspanne, innerhalb der sich der Anwender von Haarfarben in Kontakt mit dem Färbemittel befindet, reicht von der Herstellung der Anwendungsmischung über deren Auftragung auf die Haare und den Zeitraum des Einwirkens bis zum Auswaschen der Formulierung. Bei üblichen Einwirkzeiten von 30 bis 45 Minuten kann der gesamte Prozess bis zu 90 Minuten, im Höchstfall bis zu zwei Stunden Zeit in Anspruch nehmen. Eine geruchliche Abdeckung des Ammoniaks, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Ammoniak-Geruchs ist aus dem Stand der Technik bislang nicht bekannt.

Aufgabe der vorliegenden Erfindung war daher die Bereitstellung von Mitteln zum oxidativen Färben und/oder Aufhellen von Haaren mit reduziertem Ammoniakgeruch. Hierbei sollen die Mittel keine Einbußen in ihrer färberischen Leistung, insbesondere bei ihrer Grauabdeckung und ihrer Waschechtheit, aufweisen. Darüber hinaus soll die Anwendung der Mittel nicht mit einer höheren Haarschädigung verbunden sein.

Es war hierbei insbesondere die Aufgabe der vorliegenden Erfindung, über die gesamte Anwendungsdauer eine Reduktion des Ammoniakgeruchs zu erzielen. Auch nach maximal zweistündigem Kontakt sollte die geruchliche Wahrnehmung des Ammoniaks immer noch wirksam minimiert sein. Überraschenderweise hat sich im Zuge der zu dieser Erfindung führenden Arbeiten herausgestellt, dass es möglich ist, die geruchliche Wahrnehmung von Ammoniak in Mitteln zum Färben und/oder Aufhellen von keratinischen Fasern wirksam über den gesamten Anwendungszeitraum zu minimieren, wenn den Mitteln neben den Oxidationsfarbstoffen und Ammoniak eine Kombination aus speziellen, hoch ethoxylierten Fettalkoholethoxylaten und speziellen zwitterionischen Polymeren zugesetzt wird.

Ein erster Gegenstand der vorliegenden Erfindung ist daher ein Mittel zum Färben und/oder Aufhellen von keratinischen Fasern, insbesondere menschlichen Haaren, enthaltend in einem kosmetischen Träger

(a) ein oder mehrere Oxidationsfarbstoffvorprodukte,
(b) Ammoniak,
(c) ein oder mehrere ethoxylierte Fettalkohole mit einem Ethoxylierungsgrad von 80 bis 120,
(c') einen oder mehrere ethoxylierte Fettalkohole mit einem Ethoxylierungsgrad von 30,
(d) ein oder mehrere zwitterionische Polymere,

und zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol), mit der Maßgabe, dass das Mittel keine Carbonate enthält.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen

Fasern jedoch um menschliche Haare.

Unter dem erfindungsgemäß verwendeten Begriff "Mittel zum Färben und/oder Aufhellen" von Keratinfasern werden oxidative Färbemittel verstanden. Oxidative Färbemittel enthalten Oxidationsfarbstoffvorprodukte, sogenannte Entwickler und Kupplerkomponenten. Entwickler und Kuppler diffundieren getrennt in die Keratinfaser hinein und bilden unter dem Einfluss von Ammoniak als Alkalisierungsmtitel und einem Oxidationsmittel (meist Wasserstoffperoxid) in chemischer Reaktion miteinander die eigentlichen Farbstoffe aus. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört.

Je nach Einsatzmengen der Oxidationsfarbstoffvorprodukte und des Oxidationsmittel kann es sich bei der oxidativen Färbung daher vornehmlich um eine Färbung (mit hohem Farbstoffanteil) oder vornehmlich um eine Aufhellung (mit hohem Anteil an Oxidationsmittel) handeln. In letzterem Fall werden die Oxidationsfarbstoffvorprodukte hauptsächlich zur Nuancierung des Aufhellergebnisses eingesetzt.

Die erfindungsgemäßen Mittel enthalten die erfindungswesentlichen Bestandteile in einem kosmetischen Träger, bevorzugt in einem geeigneten wässrigen, alkoholischen oder wässrigalkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Als ersten wesentlichen Formulierungsbestandteil (a) enthalten die erfindungsgemäßen Mittel zum Färben und/oder Aufhellen von Keratinfasern ein oder mehrere Oxidationsfarbstoffvorprodukte. Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N’-Bis-(2-hydroxyethyl)-N,N’-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N’-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2’-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäßen Mittel ein oder mehrere Oxidationsfarbstoffvorprodukte (a) in einer Gesamtmenge von 0,01 bis 4,0 Gew.-%, bevorzugt von 0,1 bis 3,5 Gew.-%, weiter bevorzugt von 0,6 bis 3,1 Gew.-% und ganz besonders bevorzugt von 1,2 bis 2,2 Gew.-%, bezogen auf das Gesamtgewicht des anwendungsbereiten Mittels.

Als erfindungswesentliches Alkalisierungsmittel und als zweite Komponente (b) enthalten die erfindungsgemäßen Mittel weiterhin Ammoniak.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

Es hat sich herausgestellt, dass die geruchliche Wahrnehmung des Ammoniaks besonders effektiv und über einen besonders langen Zeitraum minimiert werden kann, wenn Ammoniak (b) und die weiteren wesentlichen Formulierungsbestandteile (c) und (d) in einem speziellen Mengenverhältnis zueinander stehen. Entsprechend ist es besonders bevorzugt, wenn Ammoniak in dem erfindungsgemäßen Mittel in einem bestimmten Mengenbereich eingesetzt wird.

In einer weiteren besonders bevorzugten Ausführungsform ist ein Mittel zum Färben und/oder Aufhellen von keratinischen Fasern daher dadurch gekennzeichnet, dass es Ammoniak (b) in einer Menge von 0,25 bis 1,75 Gew.-%, bevorzugt von 0,4 bis 1,4 Gew.-%, weiter bevorzugt von 0,6 bis 1,1 Gew.-% und besonders bevorzugt von 0,7 bis 0,9 Gew.-% - bezogen auf das Gesamtgewicht des anwendungsbereiten Mittels - enthält.

Die zuvor genannten bevorzugten und besonders bevorzugten Mengenangaben an Ammoniak (b) gehen von reinem Ammoniak als Berechnungsgrundlage aus. Wenn also ganz besonders bevorzugt 0,7 bis 0,9 Gew.-% Ammoniak (b) im anwendungsbereiten Mittel eingesetzt werden, so entspricht dies dem Einsatz einer Menge von 2,8 bis 3,6 g einer 25 Gew.-%igen Ammoniaklösung im anwendungsbereiten Färbemittel.

Wie bereits zuvor beschrieben, ist der vollständige oder teilweise Austausch von Ammoniak durch alternative, geruchslose Alkalisierungsmittel mit verschiedensten anwendungstechnischen Nachteilen behaftet. Insbesondere das Hinzufügen von Carbonaten ist mit einer verstärkten Haarschädigung verbunden. Es besteht daher die Maßgabe, dass die erfindungsgemäßen Mittel keine Carbonate enthalten.

Carbonate im Sinn der vorliegenden Erfindung sind alle Salze, die als Anion Carbonat oder Hydrogencarbonat enthalten. Von dieser Definition umfasst sind anorganische Carbonatsalze wie beispielsweise Natriumcarbonat, Natriumhydrogencarbonat, Kaliumcarbonat, Kaliumhydrogencarbonat, Ammoniumcarbonat, Magnesiumcarbonat oder Calciumcarbonat. Auch die Carbonatsalze von Metallen oder Übergangsmetallen liegen innerhalb der Definition der voliegenden Erfindung. Von der Definition der Carbonate ebenfalls mit umfasst sind die Salze von organischen Kationen (wie beispielsweise Tetraalkylammoniumionen), die als Gegenion ein Carbonat ($CO_3^{2-}$) oder Hydrogencarbonat-Anion ($HCO_3^-$) besitzen.

Definitionsgemäß enthält ein erfindungsgemäßes Mittel keine Carbonate, wenn der Gesamtgehalt an Carbonaten im erfindungsgemäßen Mittel 0 Gew.-% - bezogen auf das Gesamtgewicht des anwendungsbereiten Mittels - beträgt.

Auch wenn Ammoniak vollständig oder teilweise durch Alkanolamine ersetzt wird, ist dies mit anwendungstechnischen Nachteilen verbunden. Insbesondere kann der Ersatz von Ammoniak durch Alkanolamine zu einer Verschlechterung der Waschechtheiten und der Grauabdeckungen bestimmter Nuancen führen.

In einer weiteren bevorzugten Ausführungsform ist ein Mittel zum Färben und/oder Aufhellen von keratinischen Fasern deshalb dadurch gekennzeichnet, dass es keine Alkanolamine enthält.

Bei Alkanolaminen handelt es sich um primäre, sekundäre oder tertiäre Amine mit einem $C_2$-$C_6$-Alkylgrundkörper, der mindestens eine Hydroxylgruppe trägt. Beispielhaft als Alkanolamine können 2-Aminoethan-1-ol (Monoethanolamin), 2-Amino-2-methylpropan-1-ol, 2-Amino-2-methyl-propan-1,3-diol und Triethanolamin genannt werden.

Definitionsgemäß enthält ein erfindungsgemäßes Mittel keine Alkanolamine, wenn der Gesamtgehalt an Alkanolaminen im erfindungsgemäßen Mittel weniger als 0,1 Gew.-%, bevorzugt weniger als 0,05 Gew.-% besonders bevorzugt weniger als 0,01 Gew.-% und außerordentlich bevorzugt 0 Gew.-% - bezogen auf das Gesamtgewicht des anwendungsbereiten Mittels - beträgt.

Als dritten erfindungswesentlichen Formulierungsbestandteil (c) enthalten die erfindungsgemäßen Mittel zum Färben und/oder Aufhellen von Keratinfasern einen oder mehrere ethoxylierte Fettalkohole mit einem Ethoxylierungsgrad von 80 bis 120.

Unter Fettalkoholen sind erfindungsgemäß gesättigte oder ungesättigte, unverzweigte oder verzweigte $C_8$-$C_{24}$-Alkylgruppen mit Hydroxysubstitution zu verstehen. Ungesättigte Fettalkohole können einfach oder mehrfach ungesättigt sein. Bei einem ungesättigten Fettalkohol kann bzw. können deren C-C-Doppelbindung(en) die Cis- oder Trans-Konfiguration aufweisen.

Bevorzugte Fettalkohole sind Octan-1-ol (Octylalkohol, Caprylalkohol), Decan-1-ol (Decylalkohol, Caprinalkohol), Dodecan-1-ol (Dodecylalkohol, Laurylalkohol), Tetradecan-1-ol, (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Eicosan-1-ol (Eicosylalkohol, Arachylalkohol), (9*Z*)-Eicos-9-en-1-ol (Gadoleylalkohol), (5*Z*,8*Z*, 11*Z*, 14*Z*)-Eicosa-5,8, 11,14-tetraen-1-ol (Arachidonalkohol), Docosan-1-ol (Docosylalkohol, Behenylalkohol), (13*E*)-Docosen-1-ol (Brassidylalkohol) und (13*Z*)-Docos-13-en-1-ol (Erucylalkohol). Innerhalb dieser Gruppe wiederum sind Hexadecan-1-ol (Hexadecylalkohol, Cetylalkohol, Palmitylalkohol) und Octadecan-1-ol (Octadecylalkohol, Stearylalkohol) ganz besonders bevorzugt Fettalkohole.

Zur Ausbildung des erfindungswesentlichen Bestandteils (c) sind diese Fettalkohole mit einem Ethoxylierungsgrad von 80 bis 120 ethoxliert.

Unter Ethoxylierung (auch Oxyethylierung) wird die Reaktion der Fettalkohole mit Ethylenoxid (EO) verstanden. Durch Insertion von 80 bis 120 Gruppierungen des Typs -CH2-CH2-O- pro Fettalkohol Molekül entstehen lineare Polyether, die an einem Kettenende eine Hydroxygruppe und am anderen Kettenende die $C_8$-$C_{24}$-Alkylgruppe des Fettalkohols tragen.

Bevorzugte ethoxylierte Fettalkohole (c) besitzen einen Ethoxylierungsgrad von 90 bis 110. Ganz besonders bevorzugt ist es, wenn ethoxylierte Fettalkohole (c) eingesetzt werden, die einen Ethoxylierungsgrad von 100 besitzen.

In einer weiteren ganz besonders bevorzugten Ausführungsform ist ein Mittel zum Färben und/oder Aufhellen von keratinischen Fasern dadurch gekennzeichnet, dass es als ethoxylierte(n) Fettalkohol(e) (c) mit einem Ethoxylierungsgrad von 80 bis 120 eine oder mehrere Verbindungen der Formel (I) enthält,

$$R1-\left[O-CH_2-CH_2\right]_n-OH \qquad (I)$$

worin R1 für eine gesättigte oder ungesättigte, unverzweigte oder verzweigte $C_8$-$C_{24}$-Alkylgruppe, bevorzugt für eine gesättigte, unverzeigte $C_{16}$- bis $C_{18}$- Alkylgruppe, steht und n für eine ganze Zahl von 80 bis 120, bevorzugt für eine ganze Zahl von 90 bis 110 und besonders bevorzugt für die Zahl 100, steht.

Im Zuge der zu dieser Erfindung führenden Arbeiten hat sich gezeigt, dass der Ethoxylierungsgrad des ethoxylierten Fettalkohols (c) überraschenderweise die Fähigkeit des Mittels zur Reduzierung des Ammoniak-Geruchs wesentlich beeinflusst. Aus diesem Grund ist es besonders bevorzugt, wenn als ethoxylierte(r) Fettalkohol(e) (c) ein oder mehrere ethoxylierte Fettalkohole mit einem ganz bestimmten Ethoxylierungsgrad eingesetzt werden. Ganz besonders bevorzugt ist es, wenn als ethoxylierter Fettalkohol (c) ein oder mehrere ethoxylierte Fettalkohole aus der Gruppe eingesetzt werden, die in der prioritätsbegründenen Anmeldung auf den Seiten 8 bis 18 offenbart ist.

Ein ganz besonders vorteilhaftes und damit explizit ganz besonders bevorzugtes Mittel zum Färben und/oder Aufhellen von keratinischen Fasern ist dadurch gekennzeichnet, dass es als ethoxylierte(n) Fettalkohol(e) (c) eine oder mehrere Verbindungen aus der Gruppe

- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,

- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

enthält.

Wie zuvor erwähnt, kann der Ammoniakgeruch der erfindungsgemäßen Mittel zum Färben und/oder Aufhellen von Haaren insbesondere dann besonders effektiv und über einen langen Zeitraum reduziert werden, wenn die Einsatzmengen von Ammoniak (b) und der weiteren wesentlichen Formulierungsbestandteile (c) und (d) in einem speziellen Mengenverhältnis zueinander stehen. Demzufolge ist von besonderem Vorteil, wenn nicht nur der Ammoniak (b), sondern auch die ethoxylierten Fettalkohole (c) mit einem Ethoxylierungsgrad von 80 bis 120 in speziellen Mengenbereichen eingesetzt werden.

In einer weiteren bevorzugten Ausführungsform ist ein Mittel zum Färben und/oder Aufhellen von keratinischen Fasern deshalb dadurch gekennzeichnet, dass es einen oder mehrere ethoxylierte Fettalkohole (c) mit einem Ethoxylierungsgrad von 80 bis 120 in einer Gesamtmenge von 0,2 bis 1,5 Gew.-%, bevorzugt von 0,3 bis 1,2 Gew.-%, weiter bevorzugt von 0,4 bis 0,9 Gew.-% und besonders bevorzugt von 0,5 bis 0,8 Gew.-% - bezogen auf das Gesamtgewicht des anwendungsbereiten Mittels - enthält.

Als vierten erfindungswesentlichen Bestandteil enthält das erfindungsgemäße Mittel ein oder mehrere zwitterionische Polymere (d).

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Polymere werden durch Polymerisation von einem Monomertyp oder durch Polymerisation von verschiedenen, strukturell voneinander unterschiedlichen Monomertypen hergestellt. Wird das Polymer durch Polymerisation eines Monomertyps hergestellt, spricht man von Homo-Polymeren. Werden strukturell unterschiedliche Monomertypen in der Polymerisation eingesetzt, spricht der Fachmann von Copolymeren.

Das maximale Molekulargewicht des Polymers hängt vom Polymerisationsgrad (Anzahl der polymerisierten Monomere) ab und wird durch die Polymerisationsmethode mitbestimmt. Im Sinne der vorliegenden Erfindung ist es bevorzugt, wenn das maximale Molekulargewicht des zwitterionischen Polymers (d) nicht mehr als $10^7$ g/mol, bevorzugt nicht mehr als $10^6$ g/mol und besonders bevorzugt nicht mehr als $10^5$ g/mol beträgt.

Unter zwitterionischen Polymeren werden solche Polymere verstanden, die im Makromolekül sowohl kationische Gruppierungen als auch anionische Gruppierungen enthalten. Bei den im Makromolekül enthaltenen kationischen Gruppierungen handelt es sich quartäre Ammoniumgruppen. Bei diesen quartären Ammoniumgruppen trägt ein positiv geladenes Stickstoffatom vier organische Reste. Bei den anionischen Gruppierungen handelt es sich um -COO--Gruppen oder $-SO_3^-$-Gruppen.

Um eine besonders dauerhafte und starke Minimierung des Ammoniak-Geruchs zu erzielen, ist es von besonderem Vorteil, auch die zwitterionischen Polymere (d) in speziellen Mengenbereichen einzusetzen.

In einer weiteren besonders bevorzugten Ausführungsform ist ein Mittel zum Färben und/oder Aufhellen von keratinischen Fasern daher dadurch gekennzeichnet, dass es ein oder mehrere zwitterionische Polymere (d) in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.-% - bezogen auf das Gesamtgewicht des anwendungsbereiten Mittels - enthält.

Bevorzugte zwitterionische Polymere (d) werden ausgewählt aus der Gruppe der

- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminium-salzen und Acrylsäure,
- Copolymere aus N,N,N-Trimethyl-3-[(2-methyl-1-oxo-2-propen-1-yl)amino]-1-propanaminium-salzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminium-salzen, Acrylsäure und Acrylamid, z.B. Polyquaternium-53
- Copolymere aus N,N,N-Trimethyl-3-[(2-methyl-1-oxo-2-propen-1-yl)amino]-1-propanaminium-salzen, Methacrylsäure und Acrylamid,

- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten bevorzugten zwitterionischen Polymere (d) können in den erfindungsgemäßen Mittel enthalten sein.

Innerhalb der Gruppe der bevorzugten zwitterionischen Polymere (d) besitzen ganz bestimmte zwitterionische Polymere eine besonders herausragende Eignung zur Reduzierung des Ammoniak-Geruchs. Bei Einsatz dieser ausgewählten Polymere wird nicht nur die geruchliche Wahrnehmung des Färbmittels optimiert, sondern gleichzeitig auch die Waschechtheit und die Grauabdeckung der Färbemittel verbessert.

Aus diesem Grund ist in einer weiteren explizit ganz besonders bevorzugten Ausführungsform ein Mittel zum Färben und/oder Aufhellen von keratinischen Fasern dadurch gekennzeichnet, dass es ein oder mehrere zwitterionische Polymere (d) enthält, die mindestens eine anionische Struktureinheit der Formel (II) und mindestens eine kationische Struktureinheit der Formel (III) enthalten,

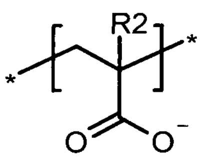

(II)

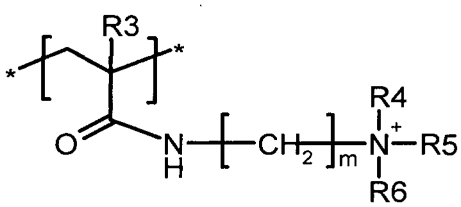

(III)

worin R2 und R3 unabhängig voneinander für ein Wasserstoffatom oder eine Methylgruppe stehen, m für eine ganze Zahl von 2 bis 6, bevorzugt für die Zahlen 2 oder 3, steht und die Reste R4, R5 und R6 unabhängig voneinander für eine $C_1$-$C_6$-Alkylgruppe, bevorzugt unabhängig voneinander für eine Methylgruppe, eine Ethylgruppe oder eine Propylgruppe, stehen.

Ein besonders bevorzugtes zwitterionisches Polymer dieses Typs ist unter dem INCI-Namen Acrylamidopropyltrimonium chloride /Acrylate Copolymer bekannt.

In einer weiteren ebenfalls besonders bevorzugten Ausführungsform ist ein erfindungsgemäßes Mittel zum Färben und/oder Aufhellen von keratinischen Fasern dadurch gekennzeichnet, dass es ein oder mehrere zwitterionische Polymere (d) enthält, die mindestene eine anionische Struktureinheit der Formel (IV) und mindestens eine kationische Struktureinheit der Formel (V) enthalten,

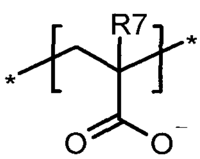

(IV)

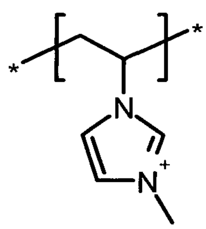

(V)

worin R7 für ein Wasserstoffatom oder eine Methylgruppe steht.

Im Zuge der zu dieser Erfindung führenden Arbeiten hat sich weiterhin gezeigt, dass die erfindungsgemäße Aufgabenstellung insbesondere dann vollständig und in zufrieden stellender Weise gelöst werden kann, wenn die erfindungsgemäßen Mittel weitere ausgewählte Formulierungsbestandteile enthalten.

So hat sich herausgestellt, dass die zusätzliche Anwesenheit von bestimmten, höherkettigen Fettalkoholen das geruchliche Ergebnis der erfindungsgemäßen Zusammensetzungen noch weiter verbessert. Daher ist es bevorzugt, wenn die erfindungsgemäßen Mittel zusätzlich einen oder mehrere Fettalkohole aus der Gruppe Arachylalkohol (Eisocan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) enthalten.

Besonders geeignete Mittel enthalten einen oder mehrere höherkettige Alkohole der vorgenannten Gruppe in einer Gesamtmenge von 1,0 bis 10,0 Gew.-%, bevorzugt von 1,4 bis 8,0 Gew.-%, weiter bevorzugt von 1,8 bis 6,0 Gew.-

% und besonders bevorzugt von 2,0 bis 4,0 Gew.-% - bezogen auf das Gesamtgewicht des anwendungsbereiten Mittels.

Ein erfindungsgemäßes Mittel ist daher dadurch gekennzeichnet, dass es zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol) in einer Gesamtmenge von 0,1 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels - enthält.

Ebenfalls von Vorteil im Hinblick auf eine maximale Reduzierung des Ammoniak-Geruchs ist die Anwesenheit eines weiteren ethoxylierten Fettalkohols (c') mit einem Ethoxylierungsgrad von 30. Ein erfindungsgemäßes Mittel zum Färben und/oder Aufhellen von keratinischen Fasern ist deshalb dadurch gekennzeichnet, dass es zusätzlich ein oder mehrere ethoxylierte Fettalkohole (c') mit einem Ethoxylierungsgrad von 30 enthält.

Geeignete Fettalkohole mit einem Ethoxylierungsgrad von 30 sind

- Dodecan-1-ol (Dodecylalkohol, Laurylalkohol), ethoxyliert mit 30 EO,
- Tetradecan-1-ol, (Tetradecylalkohol, Myristylalkohol), ethoxyliert mit 30 EO,
- Hexadecan-1-ol (Hexadecylalkohol, Cetylalkohol, Palmitylalkohol), ethoxyliert mit 30 EO,
- Hexadecan-1-ol (Hexadecylalkohol, Cetylalkohol, Palmitylalkohol), ethoxyliert mit 30 EO,
- Octadecan-1-ol (Octadecylalkohol, Stearylalkohol), ethoxyliert mit 30 EO,
- (9*Z*)-Octadec-9-en-1-ol (Oleylalkohol), ethoxyliert mit 30 EO,
- (9*E*)-Octadec-9-en-1-ol (Elaidylalkohol), ethoxyliert mit 30 EO,
- (9*Z*,12*Z*)-Octadeca-9,12-dien-1-ol (Linoleylalkohol), ethoxyliert mit 30 EO,
- Eicosan-1-ol (Eicosylalkohol, Arachylalkohol), ethoxyliert mit 30 EO,
- Docosan-1-ol (Docosylalkohol, Behenylalkohol), ethoxyliert mit 30 EO

und deren Gemische.

Entsprechend ist ein weiteres besonders bevorzugtes Mittel dadurch gekennzeichnet, dass es in einem kosmetischen Träger

(a) ein oder mehrere Oxidationsfarbstoffvorprodukte,
(b) Ammoniak,
(c) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,

(c') ein oder mehrere ethoxylierte Fettalkohole mit einem Ethoxylierungsgrad von 30, die ausgewählt sind aus der Gruppe

- Dodecan-1-ol (Dodecylalkohol, Laurylalkohol), ethoxyliert mit 30 EO,
- Tetradecan-1-ol, (Tetradecylalkohol, Myristylalkohol), ethoxyliert mit 30 EO,
- Hexadecan-1-ol (Hexadecylalkohol, Cetylalkohol, Palmitylalkohol), ethoxyliert mit 30 EO,
- Hexadecan-1-ol (Hexadecylalkohol, Cetylalkohol, Palmitylalkohol), ethoxyliert mit 30 EO,
- Octadecan-1-ol (Octadecylalkohol, Stearylalkohol), ethoxyliert mit 30 EO,
- (9*Z*)-Octadec-9-en-1-ol (Oleylalkohol), ethoxyliert mit 30 EO,
- (9*E*)-Octadec-9-en-1-ol (Elaidylalkohol), ethoxyliert mit 30 EO,
- (9*Z*,12*Z*)-Octadeca-9,12-dien-1-ol (Linoleylalkohol), ethoxyliert mit 30 EO,
- Eicosan-1-ol (Eicosylalkohol, Arachylalkohol), ethoxyliert mit 30 EO,
- Docosan-1-ol (Docosylalkohol, Behenylalkohol), ethoxyliert mit 30 EO, und

(d) ein oder mehrere zwitterionische Polymere,

und zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) enthält,

mit der Maßgabe, dass das Mittel keine Carbonate enthält.

Die ethoxylierten Fettalkohole (c) mit einem Ethoxylierungsgrad von 80 bis 120 und die ethoxylierten Fettalkohole (c') mit einem Ethoxylierungsgrad von 30 sind in einer vorteilhaften Ausführungsform in bestimmten Mengenbereichen und in speziellen Verhältnissen (c') / (c) enthalten.

In einer weiteren ebenfalls bevorzugten Ausführungsform ist ein erfindungsgemäßes Mittel zum Färben und/oder Aufhellen von keratinischen Fasern schließlich dadurch gekennzeichnet, dass es die ethoxylierten Fettalkohole(c') mit einem Ethoxlierungsgrad von 30 und die ethoxlierten Fettalkohole (c) mit einem Ethoxylierungsgrad von 80 bis 120 in einem Gewichtsverhältnis (c') / (c) von mindestens 1:1, bevorzugt in einem Gewichtsverhältnis von mindestens 1,5:1 und besonders bevorzugt in einem Gewichtsverhältnis von mindestens 2:1 - jeweils bezogen auf die Gesamtmenge aller im anwendungsbereiten Mittel enthaltenen ethoxylierten Fettalkohole (c') und die Gesamtmenge aller im anwendungsbereiten Mittel enthaltenen ethoxylierten Fettalkohole (c) - enthält.

Zusätzlich zu den vorgenannten Inhaltsstoffen können die erfindungsgemäßen Mittel weiterhin die für oxidative Färbemittel üblichen Inhaltsstoffe enthalten. Zusätzlich zu den Oxidationsfarbstoffvorprodukten können die Mittel daher auch direktziehende Farbstoffe enthalten, wobei diese direktziehenden Farbstoffe ausgewählt sein können aus den kationischen, anionischen und nichtionischen Farbstoffen.

Die Ausbildung der Farbstoffe in oxidativen Färbemitteln erfolgt erst durch den Einfluss eines Oxidationsmittels, üblicherweise wird hierfür Wasserstoffperoxid verwendet. In einer bevorzugten Ausführungsform wird das Wasserstoffperoxid als wässrige Lösung verwendet. Erfindungsgemäß bevorzugte Oxidationsmittelzubereitungen sind dadurch gekennzeichnet, dass sie 1,0 bis 23,0 Gew.- %, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges $H_2O_2$) enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Zusätzlich zu den zwitterionischen Polymeren können die erfindungsgemäßen Mittel auch anionische Polymere enthalten. Beispiele für geeignete anionische Polymere sind beispielsweise unter dem Warenzeichnen Carbopol® oder Rheothik® 11-80 im Handel erhältlich. Auch die Polymere, die unter dem INCI-Namen Acrylates Copolymere vertrieben werden, sind geeignete anionische Polymere. Ein bevorzugtes Handelsprodukt ist beispielsweise Aculyn® 33 der Firma Rohm & Haas. Weitere bevorzugte anionische Polymere werden von der Firma Rohm & Haas unter der Handelsbezeichnung Aculyn® 22 sowie von der Firma National Starch unter den Handelsbezeichnungen Structure® 2001 und Structure® 3001 vertrieben.

Geeignete zusätzlich einsetzbare kationische Polymere sind beispielsweise Polyquaternium-24 (Handelsprodukt z. B. Quatrisoft® LM 200), Copolymer 845 (Hersteller: ISP), Gaffix® VC 713 (Hersteller: ISP), Gafquat®ASCP 1011, Gafquat®HS 110, Luviquat®8155 und Luviquat® MS 370. Auch natürlich vorkommende Verdickungsmittel können auch nichtionische Guargums, wie beispielsweise sowohl modifizierte (z.B. Jaguar® HP8, Jaguar® HP60, Jaguar® HP120, Jaguar® DC 293 und Jaguar® HP105) als auch unmodifizierte Guargums (z.B. Jaguar® C) zum Einsatz kommen. Weitere geeignete Verdickungsmittel sind die Skleroglucangums oder Xanthangums, Gums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Weitere anionische, kationische oder amphotere Tenside können ebenfalls in den erfindungsgemäßen Mitteln enthalten sein. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind das N-Kokosalkylaminopropionat, das Kokosacylaminoethylaminopropionat und das $C_{12}$-$C_{18}$-Acylsarcosin.

Bevorzugte zusätzlich enthaltene kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

Ferner können die erfindungsgemäßen Mittel weitere Wirk-, Hilfs- und Zusatzstoffe enthalten, wie beispielsweise nichtionische Polymere, Silikone, kationische Polymere, Strukturanden, Lösungsmittel und Vermittler, faserstrukturverbessernde Wirkstoffe, Entschäumer wie Silikone, Antischuppenwirkstoffe, Proteinhydrolysate, pflanzliche Öle, beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl, Lichtschutzmittel, Substanzen zur Einstellung des pH-Wertes, wie beispielsweise übliche Säuren, insbesondere Genusssäuren, Vitamine, Provitamine und Vitaminvorstufen, Pflanzenextrakte, Konsistenzgeber, Wachse, weitere Quell- und Penetrationsstoffe, Perlglanzmittel wie Ethylenglykolmono- und -distearat sowie PEG-3-distearat, Treibmittel wie Propan-Butan-Gemische, $N_2O$, Dimethylether, $CO_2$ und Luft und Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Färbe- und Aufhellprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 12, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Bei den erfindungsgemäßen Mittel handelt es sich um Mittel zum oxidativen Färben und/oder Aufhellen von Haaren. Im anwendungsbereiten Mittel reagieren die Oxidationsfarbstoffvorprodukte mit dem Oxidationsmittel unter Ausbildung der eigentlichen Farbstoffe. Üblicherweise werden die erfindungsgemäßen Mittel daher als Mehrkomponentenmittel, meist als Zweikomponenten-Mittel, konfektioniert. Die erste Komponente beinhaltet hierbei die Oxidationsfarbstoffvorprodukte und das Alkalisierungsmittel, welche kurz vor der Anwendung mit einer zweiten Komponenente enthalten das Oxidationsmtitel vermischt wird. Üblicherweise werden beide Komponenten im Verhältnis 1 : 3 bis 3 : 1 miteinander vermischt. Bei dieser Mischung der Komponente enthaltend Farbcreme/Alkalisierungsmittel und der Komponente enthaltend Oxidationsmittel spricht man von der Anwendungsmischung oder dem anwendungsbereiten Mittel. Alle Mengenangaben mit Bezugnahme auf das "anwendungsbereite Mittel" beziehen sich auf die anwendungsbereite Mischung aus der Komponente enthaltend Farbcreme/Alkalisierungsmittel und der Komponente enthaltend Oxidationsmittel.

Ein weiterer Gegenstand der vorliegenden Erfindung ist die Verwendung der Kombination aus

(a) einem oder mehreren Oxidationsfarbstoffvorprodukten,
(b) Ammoniak,
(c) einem oder mehreren ethoxylierten Fettalkoholen mit einem Ethoxylierungsgrad von 80 bis 120, (c') einem oder mehreren ethoxylierten Fettalkoholen mit einem Ethoxylierungsgrad von 30,
(d) einem oder mehreren zwitterionische Polymeren,

und zusätzlich einem oder mehreren Fettalkoholen aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol),

in Mitteln zum Färben und/oder Aufhellen von Haaren zur Reduktion des Ammoniak-Geruchs vor, während und nach des Färbe- und/oder Aufhellvorgangs,
mit der Maßgabe, dass die Mittel kein Carbonate enthalten.

Bezüglich weiterer bevorzugter Ausführungsformen der erfindungsgemäßen Verfahren und Verwendungen gilt mutatis mutandis das zu den erfindungsgemäßen Mitteln Gesagte.

## Beispiele

### 1. Ausfärbungen und Bestimmung der Waschechtheiten

Es wurden die folgenden Rezepturen hergestellt.

| Formulierungsbestandteile | V1 (Gew.-%) | E1 (Gew.-%) |
|---|---|---|
| Cetylalkohol | 8,10 | 5,70 |
| Lanette 22 (INCI: Behenylalcohol) | --- | 2,40 |
| Wacker Belsil ADM 1650 (INCI: Amodimethicone) | 0,5 | 0,5 |
| Eumulgin B 1 (INCI: Ceteareth-12) | 1,2 | --- |
| Eumulgin B 3 (INCI: Ceteareth-30) | --- | 1,2 |
| Eumulgin B 2 (INCI: Ceteareth-20) | 0,6 | --- |
| Brij S 100 PA (Stearyl alcohol ethoxylated (100 EO)) | --- | 0,6 |
| Cutina GMS (INCI: Glyceryl Stearate) | 0,6 | 0,6 |
| Genamin STAC (INCI: Steartrimonium Chloride) | 1,75 | 1,75 |
| Propylene Glycol | 6,0 | 6,0 |
| p-Toluylendiamin Sulfat | 1,50 | 1,50 |
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Ethylendiamintetraessigsäure, Tetranatriumsalz | 0,20 | 0,20 |
| Natriumsulfit (wasserfrei) | 0,30 | 0,30 |
| Vitamin C | 0,05 | 0,05 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) 20 Gew.-%ige wässrige Lösung | 2,00 | 2,00 |
| Ammoniak (25 Gew.-%ige wässrige Lösung) | 5,80 | 5,80 |
| Parfum | 0,40 | 0,40 |
| Wasser | ad 100 | ad 100 |

Bei V1 handelt es sich um eine Vergleichsrezeptur, E1 ist eine erfindungsgemäße Formulierung.

Die Farbcremes wurden jeweils im Verhältnis 1:1 mit der folgenden Oxidationsmittelformierung (OX1) vermischt.

| Formulierungsbestandteile | OX1 (Gew.-%) |
|---|---|
| Phosphorsäure 85 %ig | 0,04 |
| Wasserstoffperoxid (50 %ige, wässrige Lösung) | 12,00 |
| Emulgade F (INCI: Cetearylalcohol, PEG-40 Castor Oil, Sodium Cetearyl sulfate) | 2,10 |
| Natriumbenzoat | 0,04 |
| Dinatriumpyrophosphat | 0,30 |
| Ethylendiamintetraacetat, Dinatriumsalz | 0,15 |
| Wasser | ad 100 |

Die auf diese Weise hergestellten Anwendungmischungen wurden mit einer Aplicette auf Haarsträhnen (Büffelbauchhaar) aufgetragen und dort für einen Zeitraum von 10 bis 30 Minuten belassen. Anschließend wurde die Anwendungsmischung mit einem Shampoo ausgespült und getrocknet. Dann wurden die Haarsträhnen farbmetrisch vermessen (Messung der Lab-wert)

Danach wurden die Haarsträhnen 6 mal, 12 mal und 18 mal gewaschen und jeweils nach 6, 12, und 18 Haarwäschen (HW) erneut farbmetrisch vermessen. Aus den Lab-Werten wurde jeweils der ΔE-Wert (Farbabstand) nach folgender Formel berechnet:

$$\Delta E = \sqrt{(L_0 - L_x)^2 + (a_0 - a_x)^2 + (b_0 - b_x)^2}$$

$L_0$, $a_0$, $b_0$ Farbmetrikwerte nach 0 Haarwäschen

$L_x$, $a_x$, $b_x$ Farbmetrikwerte jeweils nach 6, 12 bwz. 18 Haarwäschen

Tabelle 1: Waschechtheiten, Anwendungsdauer 10 min

| | L-Wert | a-Wert | b-Wert | ΔE | Haarwäschen (HW) |
|---|---|---|---|---|---|
| V1 + OX1 | 26,78 | 3,44 | 6,21 | --- | 0 |
| E1 + OX1 | 24,31 | 3,02 | 5,62 | --- | 0 |
| V1 + OX1 | 27,85 | 3,39 | 7,66 | 1,80 | 6 |
| E1 + OX1 | 24,57 | 3,09 | 6,55 | 0,96 | 6 |
| V1 + OX1 | 28,63 | 3,52 | 7,93 | 2,53 | 12 |
| E1 + OX1 | 25,97 | 3,23 | 7,07 | 2,22 | 12 |
| V1 + OX1 | 28,79 | 3,64 | 8,57 | 3,11 | 18 |
| E1 + OX1 | 26,23 | 3,37 | 7,55 | 2,74 | 18 |

Tabelle 2: Waschechtheiten, Anwendungsdauer 20 min

| | L-Wert | a-Wert | b-Wert | ΔE | Haarwäschen (HW) |
|---|---|---|---|---|---|
| V1 + OX1 | 18,98 | 2,79 | 3,85 | --- | 0 |
| E1 + OX1 | 18,86 | 2,47 | 3,54 | --- | 0 |
| V1 + OX1 | 19,47 | 2,88 | 5,55 | 1,77 | 6 |
| E1 + OX1 | 18,90 | 2,43 | 4,67 | 1,13 | 6 |
| V1 + OX1 | 20,34 | 2,82 | 5,31 | 2,00 | 12 |
| E1 + OX1 | 19,56 | 2,71 | 5,00 | 1,64 | 12 |
| V1 + OX1 | 19,80 | 2,82 | 5,43 | 1,78 | 18 |
| E1 + OX1 | 19,16 | 2,60 | 4,58 | 1,09 | 18 |

Tabelle 3: Waschechtheiten, Anwendungsdauer 30 min

| | L-Wert | a-Wert | b-Wert | ΔE | Haarwäschen (HW) |
|---|---|---|---|---|---|
| V1 + OX1 | 16,35 | 1,84 | 1,95 | --- | 0 |
| E1 + OX1 | 16,38 | 1,82 | 2,17 | --- | 0 |
| V1 + OX1 | 16,75 | 1,84 | 3,01 | 1,13 | 6 |
| E1 + OX1 | 16,30 | 1,80 | 2,76 | 0,60 | 6 |
| V1 + OX1 | 22,05 | 2,91 | 6,54 | 7,40 | 12 |
| E1 + OX1 | 20,42 | 2,48 | 5,00 | 4,98 | 12 |

(fortgesetzt)

| | L-Wert | a-Wert | b-Wert | ΔE | Haarwäschen (HW) |
|---|---|---|---|---|---|
| V1 + OX1 | 17,11 | 2,07 | 3,20 | 1,48 | 18 |
| E1 + OX1 | 16,57 | 2,08 | 3,03 | 0,92 | 18 |

Je größer der ΔE-Wert ist, desto größer ist der Farbabstand (Farbunterschied) der jeweiligen Strähne bei Vergleich ihrer Farbe vor und nach den Haarwäschen. Je größer der ΔE-Wert ist, desto schlechter ist damit die Waschechtheit der entsprechenden Färbung.

Es zeigte sich, dass die Waschechtheiten der mit den erfindungsgemäßen Formulierungen erhaltenen Färbungen unabhängig von der Anwendungsdauer (10 min, 20 min und 30 min) durchweg besser waren als die mit den Vergleichsformulierungen erhaltenen Färbungen.

2. Bestimmung des Ammoniakgeruchs

Die zuvor hergestellten Anwendungsmischungen (V1 + OX1, E1 + OX1) wurden jeweils auf den Kopf eines Probanden aufgetragen. Während des Anwendungszeitraums wurde der Ammoniak-Geruch von jeweils 5 geschulten Personen zu verschiedenen Zeitpunkten (direkt nach der Anwendung nach 0 min, nach 10 min, nach 20 min und nach 30 min.) bewertet. Die Bewertung erfolgte blind, was bedeutet, dass den Personen, die die Bewertung vornahmen, nicht bekannt war, welche Rezeptur sie gerade bewerteten. Aus den Einzelbewertungen wurde jeweils der Mittelwert gebildet.

Der Ammoniak-Geruch wurde auf einer Skala von 0 (quasi kein Geruch wahrnehmbar) bis 10 (sehr starker Ammoniak-Geruch) bewertet.

Tabelle 4: Ammoniak-Geruch während der Anwendung

| | nach 0 min. | nach 10 min. | nach 20 min. | nach 30 min. |
|---|---|---|---|---|
| V1+OX1 | 5 | 3,5 | 3,0 | 1,5 |
| E1+OX1 | 2,25 | 2,0 | 1,5 | 1,0 |

Es zeigt sich, dass der Ammoniak-Geruch bei Anwendung der erfindungsgemäßen Formulierung sowohl direkt nach dem Auftrag der Formulierungen als auch nach einem Zeitraum von 10 Minuten, 20 Minuten und 30 Minuten als deutlich reduziert wahrgenommen wurde.

3. Beispielformulierungen

| Formulierungsbestandteile (Farbcreme) | 1 (Gew.-%) | 2 (Gew.-%) |
|---|---|---|
| Cetylalkohol | 3,6 | 4,6 |
| Lanette 22 (INCI: Behenylalcohol) | 2,4 | 3,0 |
| Paraffinum Liquidum | 2,1 | 2,1 |
| Eumulgin B 3 (INCI: Ceteareth-30) | 1,2 | 1,8 |
| Brij S 100 PA (Stearyl alcohol ethoxylated (100 EO)) | 0,6 | 1,0 |
| Cutina GMS (INCI: Glyceryl Stearate) | 0,6 | 0,6 |
| Propylenglycol | 6,0 | 6,0 |
| p-Toluylendiamin Sulfat | 1,50 | 1,50 |
| Resorcin | 0,58 | 0,58 |
| m-Aminophenol | 0,16 | 0,16 |

(fortgesetzt)

| Formulierungsbestandteile (Farbcreme) | 1 (Gew.-%) | 2 (Gew.-%) |
|---|---|---|
| 3-Amino-2-methylamino-6-methoxypyridin | 0,05 | 0,05 |
| Kaliumhydroxid (50 %ig) | 0,7 | 0,7 |
| Ethylendiamintetraessigsäure, Tetranatriumsalz | 0,20 | 0,20 |
| Natriumsulfit (wasserfrei) | 0,30 | 0,30 |
| Vitamin C | 0,05 | 0,05 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) 20 Gew.-%ige wässrige Lösung | 3,75 | 3,0 |
| Ammoniak (25 Gew.-%ige wässrige Lösung) | 5,80 | 5,80 |
| Parfum | 0,40 | 0,40 |
| Wasser | ad 100 | ad 100 |

Die Farbcremes 1 und 2 wurden jeweils im Verhälnis 1:1 mit Oxidationsmittelformierung (OX 2) vermischt und auf Haaren ausgefärbt.

Oxidationsmittelformulierung

| Formulierungsbestandteile | OX2 (Gew.-%) |
|---|---|
| Natriumbenzoat | 0,04 |
| Dipicolinsäure (Pyridin-2,6-dicarbonsäure) | 0,10 |
| Dinatriumpyrophosphat | 0,10 |
| Kaliumhydroxid (50 %ig) | 0,19 |
| Propylenglycol | 0,50 |
| Paraffinum Liquidum | 2,00 |
| Cetearylalkohol | 3,40 |
| Ceteareth-20 | 1,00 |
| Wasserstoffperoxid (50 %ige wässrige Lösung) | 12,20 |
| Wasser | ad 100 |

Beide Anwendungsmischungen (Farbcreme 1 + OX2, Farbcreme 2 + OX2) zeichneten sich während des gesamten Anwendungszeitraums durch einen reduzierten Ammoniakgeruch aus.

Oxidationsmittelformulierungen (alle Angaben in Gew.-)

| Formulierungsbestandteile | OX3 | OX4 | OX5 | OX6 | OX7 |
|---|---|---|---|---|---|
| Natriumbenzoat | 0,04 | 0,04 | --- | --- | 0,04 |
| Dipicolinsäure | 0,1 | 0,1 | 0,1 | 0,1 | 0,1 |
| Dinatriumpyrophosphat | 0,1 | 0,1 | 0,1 | 0,03 | 0,1 |
| Kaliumhydroxid (50 %ige wässrige Lösung) | 0,19 | 0,24 | 0,23 | --- | 0,19 |
| Propylenglycol | 0,5 | --- | -- | 4,0 | 1,0 |
| 1-Hydroxyethan-1,1-diphosphonsäure (Etidronsäure, 60 %ige wässrige Lsg.) | 0,25 | 0,31 | 0,25 | --- | 0,25 |

(fortgesetzt)

| Formulierungsbestandteile | OX3 | OX4 | OX5 | OX6 | OX7 |
|---|---|---|---|---|---|
| Paraffinum Liquidum | 2,0 | 20 | --- | --- | 0,3 |
| Cetearylalkohol | 3,6 | ---- | 0,5 | --- | 3,4 |
| Ceteareth-20 | 1,2 | --- | 0,5 | --- | 1,0 |
| Wasserstoffperoxid (50 %ige wässrige Lösung | 6,2 | 18,2 | 23,2 | 12,2 | 12,2 |
| Emulgade F [1] | --- | --- | 4,0 | --- | --- |
| Bienenwachs | --- | --- | 0,3 | --- | --- |
| Isopropylmyristat | --- | --- | 10 | --- | --- |
| Natriumhydroxid (45 %ige wässrige Lsg) | --- | --- | --- | 0,73 | --- |
| Xanthan Gum (Keltrol CG-SFT) | --- | --- | --- | 2,0 | --- |
| Trimethylstearylammoniumchloride | --- | --- | --- | --- | 0,29 |
| Wasser | ad 100 | ad 100 | ad 100 | ad 100 | ad 100 |
| [1] Emulgade F: Cetearylalkohol, PEG-40 Castor Oil, Natriumcetearylsulfat (BASF) | | | | | |

Es wurden die folgenden Anwendungsmischungen hergestellt:
Farbcreme 1 + OX3, Farbcreme 1 + OX4, Farbcreme 1 + OX5, Farbcreme 1 + OX6, Farbcreme 1 + OX7, Farbcreme 2 + OX3, Farbcreme 2 + OX4, Farbcreme 2 + OX5 und Farbcreme 2 + OX6 und Farbcreme 2 + OX7

Hierbei erfolgte die Herstellung der Anwendungsmischung jeweils durch Vermischen der Farbcreme mit der Oxidationsmittelformulierung im Gewichtsverhältnis 1:1. Die auf diese Weise hergestellten Anwendungsmischungen wurden auf Haaren ausgefärbt.

Oxidationsmittelformulierungen (alle Angaben in Gew.-)

| Formulierungsbestandteile | OX8 | OX9 | OX10 | OX11 |
|---|---|---|---|---|
| Dipicolinsäure | 0,1 | 0,1 | 0,1 | 0,1 |
| Dinatriumpyrophosphat | 0,03 | 0,03 | 0,03 | 0,03 |
| 1-Hydroxyethan-1,1-diphosphonsäure (Etidronsäure, 60 %ige wässrige Lsg.) | 1,5 | 1,5 | 1,5 | 1,5 |
| Ceteareth-20 | 1,0 | --- | --- | --- |
| Wasserstoffperoxid (50 %ige wässrige Lösung | 20 | 12 | 10 | 12 |
| Natriumhydroxid (45 %ige wässrige Lsg) | 0,8 | --- | 0,73 | 0,83 |
| Cetylalkohol | 3,5 | --- | --- | --- |
| Disponil FES 77 [2] | 2,5 | --- | --- | --- |
| Aculyn 33 A [3] | 10 | 15 | --- | 15 |
| Ammoniak (25 %ige wässrige Lsg.) | --- | 0,65 | --- | --- |
| Natriumlaurethsulfat (27 %ige wässrige Lösung) | --- | 2,0 | --- | 2,0 |
| Dimethicone (10 %ig) | --- | 0,067 | --- | 0,067 |
| Plantacare 818 UP [4] | --- | --- | 15 | --- |
| Cremophor CO 60[5] | --- | --- | 15 | --- |
| Eumulgin L [6] | --- | --- | 0,4 | --- |
| Polyquaternium-6 | --- | --- | 0,125 | --- |
| Polyquaternium-22 (Merquat 281) | --- | --- | 1,0 | --- |
| Fruitapone Strawberry B [7] | --- | --- | 0,45 | --- |

(fortgesetzt)

| Formulierungsbestandteile | OX8 | OX9 | OX10 | OX11 |
|---|---|---|---|---|
| Isopropylmyristat | --- | --- | --- | 12,16 |
| Parfum | --- | --- | 0,2 | --- |
| Betacarotin | --- | --- | --- | 0,125 |
| Marula Öl | --- | --- | --- | 0,125 |
| Controx KS C[8] | --- | --- | --- | 0,09 |
| Wasser | ad 100 | ad 100 | ad 100 | ad 100 |
| [2]Disponil FES 77: INCI SODIUM COCETH-30 SULFATE (32-34 %ige wässrige Lösung, BASF)<br>[3]Aculyn 33 A: INCI Acrylates Copolymer (27 - 29 %ige wässrige Lösung, Rohm und Haas)<br>[4]Plantacare 818 UP: C8-16 Alkylpolyglucoside (30 - 50 %ige wässrige Lösung, BASF)<br>[5]Cremophor CO 60: INCI PEG-60 hydrogenated castor oil (BASF)<br>[6]Eumulgin L: INCI PPG-1-PEG-9 Laurylglycolether (BASF)<br>[7]Fruitapone Strawberry B: INCI Propylene Glycol, Aqua (Water), Citric Acid, Fragaria Ananassa (Strawberry) Fruit Juice, Trideceth-9, Bisabolol (Symrise)<br>[8]Controx KS C: INCI Tocopherol, Hydrogenated Palm Glycerides Citrate (BASF) | | | | |

Es wurden die folgenden Anwendungsmischungen hergestellt:
Farbcreme 1 + OX8, Farbcreme 1 + OX9, Farbcreme 1 + OX10, Farbcreme 1 + OX11, Farbcreme 2 + OX8, Farbcreme 2 + OX9, Farbcreme 2 + OX10 und Farbcreme 2 + OX11

Hierbei erfolgte die Herstellung der Anwendungsmischung jeweils durch Vermischen der Farbcreme mit der Oxidationsmittelformulierung im Gewichtsverhältnis 1:1. Die auf diese Weise hergestellten Anwendungsmischungen wurden auf Haaren ausgefärbt.

## Patentansprüche

1. Mittel zum Färben und/oder Aufhellen von keratinischen Fasern, insbesondere menschlichen Haaren, enthaltend in einem kosmetischen Träger

   (a) ein oder mehrere Oxidationsfarbstoffvorprodukte,
   (b) Ammoniak,
   (c) einen oder mehrere ethoxylierte Fettalkohole mit einem Ethoxylierungsgrad von 80 bis 120,
   (c') einen oder mehrere ethoxylierte Fettalkohole mit einem Ethoxylierungsgrad von 30,
   (d) ein oder mehrere zwitterionische Polymere,

   und zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol)
   mit der Maßgabe, dass das Mittel keine Carbonate enthält.

2. Mittel nach Anspruch 1, **dadurch gekennzeichnet, dass** es Ammoniak (b) in einer Menge von 0,25 bis 1,75 Gew.-%, bevorzugt von 0,4 bis 1,4 Gew.-%, weiter bevorzugt von 0,6 bis 1,1 Gew.-% und besonders bevorzugt von 0,7 bis 0,9 Gew.-% - bezogen auf das Gesamtgewicht des anwendungsbereiten Mittels - enthält.

3. Mittel nach einem der Ansprüche 1 oder 2, **dadurch gekennzeichnet, dass** es als ethoxylierte(n) Fettalkohol(e) (c) mit einem Ethoxylierungsgrad von 80 bis 120 eine oder mehrere Verbindungen der Formel (I) enthält,

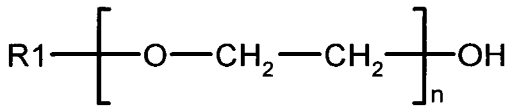

(I)

worin R1 für eine gesättigte oder ungesättigte, unverzweigte oder verzweigte $C_8$-$C_{24}$-Alkylgruppe, bevorzugt für eine gesättigte, unverzweigte $C_{16}$- oder $C_{18}$- Alkylgruppe, steht und
n für eine ganze Zahl von 80 bis 120, bevorzugt für eine ganze Zahl von 90 bis 110 und besonders bevorzugt für die Zahl 100, steht.

**4.** Mittel nach einem der Ansprüche 1 bis 3, **dadurch gekennzeichnet, dass** es einen oder mehrere ethoxylierte Fettalkohole (c) mit einem Ethoxylierungsgrad von 80 bis 120 in einer Gesamtmenge von 0,2 bis 1,5 Gew.-%, bevorzugt von 0,3 bis 1,2 Gew.-%, weiter bevorzugt von 0,4 bis 0,9 Gew.-% und besonders bevorzugt von 0,5 bis 0,8 Gew.-% - bezogen auf das Gesamtgewicht des anwendungsbereiten Mittels - enthält.

**5.** Mittel nach einem der Ansprüche 1 bis 4, **dadurch gekennzeichnet, dass** es ein oder mehrere zwitterionische Polymere (d) in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.- % - bezogen auf das Gesamtgewicht des anwendungsbereiten Mittels - enthält.

**6.** Mittel nach einem der Ansprüche 1 bis 5, **dadurch gekennzeichnet, dass** es ein oder mehrere zwitterionische Polymere (d) enthält, die mindestens eine anionische Struktureinheit der Formel (II) und mindestens eine kationische Struktureinheit der Formel (III) enthalten,

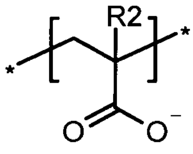

(II)

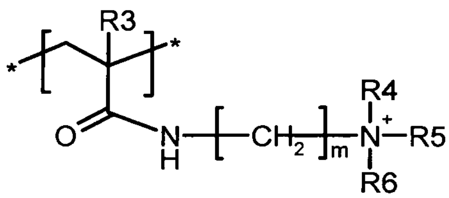

(III)

worin R2 und R3 unabhängig voneinander für ein Wasserstoffatom oder eine Methylgruppe stehen, m für eine ganze Zahl von 2 bis 6, bevorzugt für die Zahlen 2 oder 3, steht und
die Reste R4, R5 und R6 unabhängig voneinander für eine $C_1$-$C_6$-Alkylgruppe, bevorzugt unabhängig voneinander für eine Methylgruppe, eine Ethylgruppe oder eine Propylgruppe, stehen.

**7.** Mittel nach einem der Ansprüche 1 bis 6, **dadurch gekennzeichnet, dass** es zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,1 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels - enthält.

**8.** Mittel nach einem der Ansprüche 1 bis 7, **dadurch gekennzeichnet, dass** es die ethoxylierten Fettalkohole (c') mit einem Ethoxlierungsgrad von 30 und die ethoxlierten Fettalkohole (c) mit einem Ethoxylierungsgrad von 80 bis 120 in einem Gewichtsverhältnis (c') / (c) von mindestens 1:1, bevorzugt in einem Gewichtsverhältnis von mindestens 1,5:1 und besonders bevorzugt in einem Gewichtsverhältnis von mindestens 2:1 - jeweils bezogen auf die Gesamtmenge aller im anwendungsbereiten Mittel enthaltenen ethoxylierten Fettalkohole (c') und die Gesamtmenge aller im anwendungsbereiten Mittel enthaltenen ethoxylierten Fettalkohole (c) - enthält.

**9.** Verwendung der Kombination aus

(a) einem oder mehreren Oxidationsfarbstoffvorprodukten,
(b) Ammoniak,
(c) einem oder mehreren ethoxylierten Fettalkoholen mit einem Ethoxylierungsgrad von 80 bis 120

(c') einem oder mehreren ethoxylierten Fettalkoholen mit einem Ethoxylierungsgrad von 30,
(d) einem oder mehreren zwitterionischen Polymeren,

und zusätzlich einem oder mehreren Fettalkoholen aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol),
in Mitteln zum Färben und/oder Aufhellen von Haaren zur Reduktion des Ammoniak-Geruchs vor, während und nach dem Färbe- und/oder Aufhellvorgang, mit der Maßgabe, dass die Mittel kein Carbonate enthalten.

## Claims

**1.** An agent for dyeing and/or lightening keratin fibers, in particular human hair, containing, in a cosmetic carrier:

(a) one or more oxidation dye precursors,
(b) ammonia,
(c) one or more ethoxylated fatty alcohols having a degree of ethoxylation of from 80 to 120,
(c') one or more ethoxylated fatty alcohols having a degree of ethoxylation of 30,
(d) one or more zwitterionic polymers,

and additionally one or more fatty alcohols from the group of arachidyl alcohol (eicosan-1-ol), gadoleyl alcohol ((9Z)-eicos-9-en-1-ol), arachidonyl alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol) and brassidyl alcohol ((13*E*)-docosen-1-ol), with the proviso that the agent does not contain carbonates.

**2.** The agent according to claim 1, **characterized in that** it contains ammonia (b) in an amount of from 0.25 to 1.75 wt.%, preferably from 0.4 to 1.4 wt.%, more preferably from 0.6 to 1.1 wt.% and particularly preferably from 0.7 to 0.9 wt.%, based on the total weight of the ready-to-apply agent.

**3.** The agent according to one of claims 1 or 2, **characterized in that** it contains one or more compounds of formula (I) as ethoxylated fatty alcohol(s) (c) having a degree of ethoxylation of from 80 to 120,

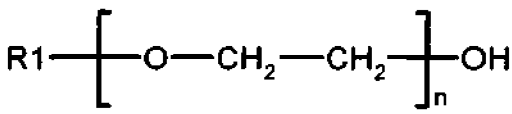

(I)

where R1 represents a saturated or unsaturated, unbranched or branched $C_8$-$C_{24}$ alkyl group, preferably a saturated, unbranched $C_{16}$ or $C_{18}$ alkyl group, and n represents an integer of from 80 to 120, preferably an integer from 90 to 110, and particularly preferably the number 100.

**4.** The agent according to one of claims 1 to 3, **characterized in that** it contains one or more ethoxylated fatty alcohols (c) having a degree of ethoxylation of from 80 to 120 in a total amount of from 0.2 to 1.5 wt.%, preferably from 0.3 to 1.2 wt.%, more preferably from 0.4 to 0.9 wt.% and particularly preferably from 0.5 to 0.8 wt.%, based on the total weight of the ready-to-apply agent.

**5.** The agent according to one of claims 1 to 4, **characterized in that** it contains one or more zwitterionic polymers (d) in a total amount of from 0.1 to 1.5 wt.%, preferably from 0.2 to 1.2 wt.%, more preferably from 0.3 to 0.8 wt.% and particularly preferably from 0.4 to 0.6 wt.%, based on the total weight of the ready-to-apply agent.

**6.** The agent according to one of claims 1 to 5, **characterized in that** it contains one or more zwitterionic polymers (d) containing at least one anionic structural unit of formula (II) and at least one cationic structural unit of formula (III),

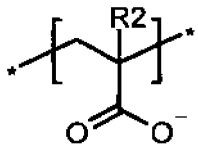

(II)

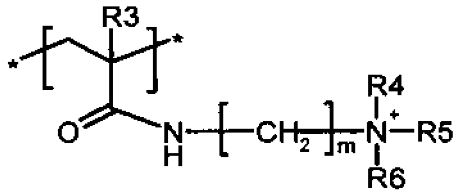

(III)

where R2 and R3 represent, independently of one another, a hydrogen atom or a methyl group, m represents an integer of from 2 to 6, preferably the numbers 2 or 3, and the functional groups R4, R5 and R6 represent, independently of one another, a $C_1$-$C_6$ alkyl group, and preferably represent, independently of one another, a methyl group, an ethyl group or a propyl group.

7. The agent according to one of claims 1 to 6, **characterized in that** it additionally contains one or more fatty alcohols from the group of arachidyl alcohol (eicosan-1-ol), gadoleyl alcohol ((9Z)-eicos-9-en-1-ol), arachidonyl alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), and brassidyl alcohol ((13*E*)-docosen-1-ol) in a total amount of from 0.1 to 10.0 wt.%, preferably from 1.4 to 8.0 wt.%, more preferably from 1.8 to 6.0 wt.% and particularly preferably from 2.0 to 4.0 wt.%, based on the total weight of the ready-to-apply agent.

8. The agent according to one of claims 1 to 7, **characterized in that** it contains the ethoxylated fatty alcohols (c') having a degree of ethoxylation of 30 and the ethoxylated fatty alcohols (c) having a degree of ethoxylation of from 80 to 120 in a weight ratio (c')/(c) of at least 1:1, preferably in a weight ratio of at least 1.5:1, and particularly preferably in a weight ratio of at least 2:1, in each case based on the total amount of all ethoxylated fatty alcohols (c') contained in the ready-to-apply agent and the total amount of all ethoxylated fatty alcohols (c) contained in the ready-to-apply agent.

9. The use of the combination of

(a) one or more oxidation dye precursors,
(b) ammonia,
(c) one or more ethoxylated fatty alcohols having a degree of ethoxylation of from 80 to 120,
(c') one or more ethoxylated fatty alcohols having a degree of ethoxylation of 30,
(d) one or more zwitterionic polymers,

and additionally one or more fatty alcohols from the group of arachidyl alcohol (eicosan-1-ol), gadoleyl alcohol ((9Z)-eicos-9-en-1-ol), arachidonyl alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol) and brassidyl alcohol ((13*E*)-docosen-1-ol), in agents for dyeing and/or lightening hair in order to reduce the ammonia odor before, during and after the dyeing and/or lightening process, with the proviso that the agents do not contain carbonates.

**Revendications**

1. Agent de coloration et/ou d'éclaircissement de fibres kératiniques, en particulier de cheveux humains, contenant, dans un support cosmétique,

(a) un ou plusieurs précurseurs de colorant d'oxydation,
(b) de l'ammoniac,
(c) un ou plusieurs alcools gras éthoxylés ayant un degré d'éthoxylation de 80 à 120,
(c') un ou plusieurs alcools gras éthoxylés ayant un degré d'éthoxylation de 30,
(d) un ou plusieurs polymères zwitterioniques,

et en outre un ou plusieurs alcools gras du groupe constitué de l'alcool arachylique (eicosan-1-ol), de l'alcool gadoléylique ([9Z]-eicos-9-en-1-ol), de l'alcool arachidonique ([5Z, 8Z, 11Z, 14Z]-eicosa-5,8,11,14-tétraène-1-ol), de l'alcool hénéicosylique (hénéicosan-1-ol), de l'alcool béhénylique (docosan-1-ol), de l'alcool érucylique ([13Z]-docos-13-én-1-ol) et de l'alcool brassidylique ([13*E*] -docosén-1-ol) à condition que l'agent ne contienne pas de carbonates.

2. Agent selon la revendication 1, **caractérisé en ce qu'**il contient de l'ammoniac (b) en une quantité de 0,25 à 1,75 % en poids, de préférence de 0,4 à 1,4 % en poids, de manière davantage préférée de 0,6 à 1,1 % en poids et de manière particulièrement préférée de 0,7 à 0,9 % en poids, par rapport au poids total de l'agent prêt à l'emploi.

3. Agent selon l'une quelconque des revendications 1 ou 2, **caractérisé en ce qu'**il contient, comme alcool(s) gras éthoxylé(s) (c) avec un degré d'éthoxylation de 80 à 120, un ou plusieurs composés répondant à la formule (I)

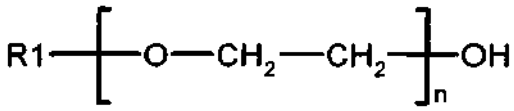

(I)

dans laquelle R1 représente groupe alkyle en $C_8$-$C_{24}$ saturé ou insaturé, non ramifié ou ramifié, de préférence un groupe alkyle en $C_{16}$ ou $C_{18}$ saturé, non ramifié, et n représente un nombre entier situé dans la plage allant de 80 à 120, de préférence un nombre entier situé dans la plage allant de 90 à 110, et de manière particulièrement préférée un nombre de 100.

4. Agent selon l'une quelconque des revendications 1 à 3, **caractérisé en ce qu'**il contient un ou plusieurs alcools gras éthoxylés (c) avec un degré d'éthoxylation de 80 à 120 en une quantité totale de 0,2 à 1,5 % en poids, de préférence de 0,3 à 1,2 % en poids, de manière davantage préférée de 0,4 à 0,9 % en poids et de manière particulièrement préférée de 0,5 à 0,8 % en poids, par rapport au poids total de l'agent prêt à l'emploi.

5. Agent selon l'une quelconque des revendications 1 à 4, **caractérisé en ce qu'**il contient un ou plusieurs polymères zwitterioniques (d) en une quantité totale de 0,1 à 1,5 % en poids, de préférence de 0,2 à 1,2 % en poids, de manière davantage préférée de 0,3 à 0,8 % en poids et de manière particulièrement préférée de 0,4 à 0,6 % en poids, par rapport au poids total de l'agent prêt à l'emploi.

6. Agent selon l'une quelconque des revendications 1 à 5, **caractérisé en ce qu'**il contient un ou plusieurs polymères zwitterioniques (d) qui contiennent au moins une unité structurelle anionique de formule (II) et au moins une unité structurelle cationique de formule (III),

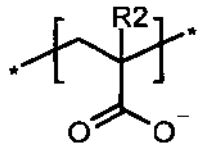

(II)

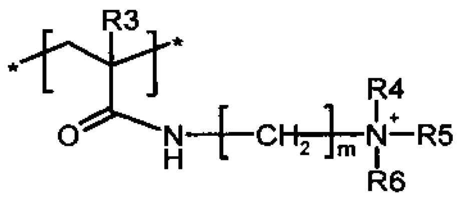

(III)

dans laquelle R2 et R3, indépendamment l'un de l'autre, représentent un atome d'hydrogène ou un groupe méthyle, m représente un entier de 2 à 6, de préférence 2 ou 3, et les radicaux R4, R5 et R6 représentent, indépendamment les uns des autres, un groupe alkyle en $C_1$-$C_6$, de préférence indépendamment les uns des autres un groupe méthyle, un groupe éthyle ou un groupe propyle.

7. Agent selon l'une des revendications 1 à 6, **caractérisé en ce qu'**il contient en outre un ou plusieurs alcools gras du groupe constitué de l'alcool arachylique (eicosan-1-ol), de l'alcool gadoléylique ((9Z)-eicos-9-en-1-ol), de l'alcool arachidonique ((5Z,8Z,11Z,14Z)-eicosa-5,8,11,14-tétraène-1-ol), de l'alcool hénicosylique (hénicosan-1-ol), de l'alcool béhénylique (docosan-1-ol), de l'alcool érucylique ((13Z)-docos-13-en-1-ol) et de l'alcool brassidylique ((13*E*)-docosen-1-ol) en une quantité totale de 0,1 à 10,0 % en poids, de préférence de 1,4 à 8,0 % en poids, de manière davantage préférée de 1,8 à 6,0 % en poids et de manière particulièrement préférée de 2,0 à 4,0 % en poids, par rapport au poids total de l'agent prêt à l'emploi.

8. Agent selon l'une quelconque des revendications 1 à 7, **caractérisé en ce qu'**il contient les alcools gras éthoxylés (c') avec un degré d'éthoxylation de 30 et les alcools gras éthoxylés (c) avec un degré d'éthoxylation de 80 à 120

dans un rapport pondéral (c')/(c) d'au moins 1:1, de préférence dans un rapport pondéral d'au moins 1,5:1 et de manière particulièrement préférée dans un rapport pondéral d'au moins 2:1, dans chaque cas par rapport à la quantité totale de tous les alcools gras éthoxylés (c') contenus dans la composition prête à l'emploi et à la quantité totale de tous les alcools gras éthoxylés (c) contenus dans l'agent prêt à l'emploi.

**9.** Utilisation de la combinaison

(a) d'un ou de plusieurs précurseurs de colorants d'oxydation,
(b) d'ammoniac,
(c) d'un ou de plusieurs alcools gras éthoxylés ayant un degré d'éthoxylation de 80 à 120
(c') d'un ou de plusieurs alcools gras éthoxylés ayant un degré d'éthoxylation de 30,
(d) d'un ou de plusieurs polymères zwitterioniques,

et en outre d'un ou de plusieurs alcools gras du groupe constitué de l'alcool arachylique (eicosan-1-ol), de l'alcool gadoléylique ((9Z)-eicos-9-én-1-ol), de l'alcool arachidonique ((5Z, 8Z, 11Z, 14Z)-eicosa-5,8,11,14-tétraén-1-ol), de l'alcool héneicosylique (heneicosan-1-ol), de l'alcool béhénylique (docosan-1-ol), l'alcool érucylique ((13Z)-docos-13-én-1-ol) et de l'alcool brassidylique ((13*E*)-docosén-1-ol), dans des compositions pour la coloration et/ou l'éclaircissement des cheveux pour réduire l'odeur d'ammoniac avant, pendant et après le processus de coloration et/ou d'éclaircissement, à condition que les compositions ne contiennent pas de carbonates.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006060570 A
- WO 2006060565 A
- WO 2005110499 A
- JP 2007191459 B
- JP 2003040750 B
- EP 1878469 A1


### In der Beschreibung aufgeführte Nicht-Patentliteratur


- **Z. B. KH. SCHRADER.** Grundlagen und Rezepturen der Kosmetika. Hüthig Buch Verlag, 1989